# EUROPÄISCHE PATENTSCHRIFT

(11) **EP 3 820 542 B1**
(45) Veröffentlichungstag und Bekanntmachung des Hinweises auf die Patenterteilung: **28.08.2024**
(21) Anmeldenummer: 19739960.3
(22) Anmeldetag: 09.07.2019
(51) Int. Cl.: A61M 1/34, A61M 1/36

(54) **STEUERUNGS- UND/ODER REGELUNGSEINRICHTUNG ZUM ENTFERNEN VON FLUID AUS EINEM BLUTFILTER**
CONTROL AND/OR REGULATING DEVICE FOR REMOVING FLUID FROM A BLOOD FILTER
DISPOSITIF DE COMMANDE ET/OU DE RÉGULATION PERMETTANT D'ÉLIMINER UN FLUIDE CONTENU DANS UN FILTRE SANGUIN

(30) Priorität: 11.07.2018 DE 102018116806
(43) Veröffentlichungstag der Anmeldung: 19.05.2021
(73) Patentinhaber: Fresenius Medical Care Deutschland GmbH, 61352 Bad Homburg (DE)
(72) Erfinder: THYS, Martin, 97508 Grettstadt (DE)
(74) Vertreter: Bobbert & Partner Patentanwälte PartmbB
(86) Internationale Anmeldenummer: PCT/EP2019/068403
(87) Internationale Veröffentlichungsnummer: WO 2020/011784

(56) Entgegenhaltungen:
- DE-A1- 102006 012 087
- DE-A1- 102014 109 136
- US-A- 5 893 382

## Beschreibung

Die vorliegende Erfindung betrifft eine Steuerungs- und/oder Regelungseinrichtung gemäß dem Oberbegriff des Anspruchs 1, konfiguriert, um ein Verfahren zum Entfernen von Fluid aus einem Blutfilter für die extrakorporale Blutbehandlung eines Patienten nach Beendigung der Blutbehandlungssitzung zu bewirken. Sie betrifft ferner eine medizinische Behandlungsvorrichtung gemäß dem Oberbegriff des Anspruchs 8. Des Weiteren betrifft die vorliegende Erfindung ein digitales Speichermedium gemäß Anspruch 10, ein Computerprogramm-Produkt gemäß Anspruch 11 sowie ein Computerprogramm gemäß Anspruch 12.

Blutfilter und extrakorporale Blutkreisläufe sind üblicherweise Einwegartikel und werden nach ihrer Verwendung entsorgt. Die Entsorgung ist kostenintensiv und nach Abfallgewicht zu vergüten. Aus diesem Grund, und auch zur Verringerung einer Kontaminationsgefahr, werden diese Einwegartikel daher vor ihrer Entsorgung von Fluid, und insbesondere von Blut, entleert.

Die DE 10 2014 109136 A1 offenbart ein Verfahren zum Entfernen von Fluid aus einem Blutfilter nach Beendigung einer Blutbehandlungssitzung mittels Flusserhöhung, und eine Behandlungsvorrichtung zum Durchführen desselben.

In der US 5 893 382 A sind ein Verfahren und eine Einrichtung zum Spülen einer Membranvorrichtung offenbart.

Aus der DE 10 2006 012087 A1 sind ein Verfahren zum zumindest teilweisen Entleeren eines extrakorporalen Blutkreislaufs und ein Hämodialysegerät zur Anwendung des Verfahrens bekannt.

Es ist Aufgabe der vorliegenden Erfindung, eine weitere Steuerungs- und/oder Regelungseinrichtung anzugeben, um ein Verfahren zum Entfernen von Fluid aus einem Blutfilter oder einer Blutkammer hiervon und ggf. aus einem hiermit verbundenen extrakorporalen Blutkreislauf nach Beendigung einer Blutbehandlung oder einer Blutbehandlungssitzung zu bewirken. Außerdem sollen dazu geeignete Vorrichtungen angegeben werden.

Die erfindungsgemäße Aufgabe wird durch eine Steuerungs- und/oder Regelungseinrichtung mit den Merkmalen des Anspruchs 1 gelöst. Sie wird ferner gelöst mittels der medizinischen Behandlungsvorrichtung mit den Merkmalen des Anspruchs 8, des digitalen Speichermediums mit den Merkmalen des Anspruchs 10, des Computerprogramm-Produkts mit den Merkmalen des Anspruchs 11 sowie des Computerprogramms mit den Merkmalen des Anspruchs 12.

Erfindungsgemäß wird somit eine Steuerungs- und/oder Regelungseinrichtung vorgeschlagen, welche ein Entfernen von Fluid, insbesondere Blut, oder einem Anteil hiervon aus einem zur Blutbehandlung eines Patienten verwendeten extrakorporalen Blutkreislauf nach Beendigung der Blutbehandlungssitzung bewirkt.

Der extrakorporale Blutkreislauf, aus dem durch Steuerung und/oder Regelung durch die erfindungsgemäße Steuerungs- und/oder Regelungseinrichtung Fluid entfernt werden kann, weist einen Blutfilter auf oder ist hiermit in Fluidkommunikation verbunden. Der Blutfilter weist wiederum eine Blutkammer und eine Dialysatkammer auf, zwischen welchen eine zumeist semi-permeable Membran angeordnet ist. Die Blutkammer ist zur Blutbehandlung mit einer zur Blutkammer hinführenden arteriellen Blutleitung (d. h. mit der Blutentnahmeleitung; diese Begriffe sind hierin austauschbar verwendet) und einer von der Blutkammer wegführenden venösen Blutleitung (d. h. mit einer Blutrückgabeleitung; diese Begriffe sind hierin austauschbar verwendet) verbunden. Die Dialysatkammer ist mit einer zu ihr hinführenden Dialysierflüssigkeitszulaufleitung und mit einer von ihr wegführenden Dialysatablaufleitung verbunden. Des Weiteren weist der extrakorporale Blutkreislauf ein Ventil auf, welches dem selektiven Herstellen oder Unterbinden einer Fluidverbindung zwischen dem Inneren des extrakorporalen Blutkreislaufs und dessen Äußeren bzw. der Atmosphäre dient.

Die medizinische Behandlungsvorrichtung, auf die die erfindungsgemäße Steuerungs- und/oder Regelungseinrichtung einwirkt, weist wenigstens eine an oder in dem extrakorporalen Blutkreislauf angeordnete Blutpumpe zum Fördern von Blut innerhalb des Leitungsinneren des extrakorporalen Blutkreislaufs auf. Außerdem umfasst sie wenigstens eine Fördereinrichtung, die dem Einbringen von Substituat oder Dialysierflüssigkeit in die arterielle Blutentnahmeleitung oder in die venöse Blutrückgabeleitung dient. Die medizinische Behandlungsvorrichtung weist ferner wenigstens eine venöse Patientenschlauchklemme, und/oder eine Betätigungseinrichtung hierfür, auf, mit welcher ein Fluss durch einen Abschnitt der venösen Blutrückgabeleitung unterbunden werden kann. Des Weiteren umfasst sie eine Betätigungseinrichtung, konfiguriert, um das oben beschriebene Ventil des extrakorporalen Blutkreislaufs zu betätigen, insbesondere zu öffnen oder zu schließen (wodurch ein Fluss unterbunden werden kann). Wenigstens ein Drucksensor, angeordnet zum Messen des in der arteriellen Blutentnahmeleitung oder der venösen Blutrückgabeleitung herrschenden Drucks, ist ebenfalls von der medizinischen Behandlungsvorrichtung umfasst.

Das Verfahren, das mittels der erfindungsgemäßen Steuerungs- und/oder Regelungseinrichtung bewirkt wird, umfasst das Zuführen von Substituat (alternativ:
Substitutionsflüssigkeit) oder Dialysierflüssigkeit zum Verdrängen des Fluids, insbesondere des Blutes, aus dem extrakorporalen Blutkreislauf, insbesondere aus dem Blutfilter und aus dessen Blutkammer. Das Verfahren umfasst die folgenden Schritte:
a) Unterbinden einer Verbindung zwischen der Atmosphäre bzw. dem Äußeren einerseits und einem Inneren des extrakorporalen Blutkreislaufs andererseits durch Schließen des oben diskutierten Ventils;
b) Schließen der venösen Patientenschlauchklemme;
c) Einbringen von Substituat oder Dialysierflüssigkeit in den extrakorporalen Blutkreislauf, insbesondere in die arterielle Blutentnahmeleitung oder in die venöse Blutrückgabeleitung, und/oder Aufbauen eines erhöhten Drucks mittels der zum Einbringen verwendeten Fördereinrichtung;
d) Optional: Messen des in der arteriellen Blutentnahmeleitung und/oder in der venösen Blutrückgabeleitung herrschenden Drucks, z. B. mittels des vorgenannten Drucksensors;
e) Herstellen der Fluidverbindung zwischen der Atmosphäre bzw. dem Äußeren und dem Inneren des Blutkreislaufs durch Öffnen des oben genannten Ventils, falls der gemessene Druck einen vorbestimmten ersten Wert erreicht oder übersteigt, falls dieser in Schritt d) gemessen wurde, oder wenn seit Beginn des Einbringens von Substituat oder seit Beginn des Aufbauens von Druck bei geschlossener venöser Patientenschlauchklemme eine vorbestimmte, erste Zeitdauer (sog. "Zeitregelung") verstrichen ist; und
f) Unterbinden der Fluidverbindung zwischen der Atmosphäre/dem Äußeren einerseits und einem Inneren des Blutkreislaufs andererseits durch Schließen des Ventils und Öffnen der Patientenschlauchklemme, beides wenn, das heißt z. B. nachdem oder sobald, der gemessene Druck auf einen vorbestimmten zweiten Wert abgefallen ist, falls dieser in Schritt d) gemessen wurde, oder wenn, d. h. nachdem, seit dem Öffnen des Ventils in Schritt e) eine vorbestimmte, zweite Zeitdauer verstrichen ist.

Die erfindungsgemäße medizinische Behandlungsvorrichtung (im Folgenden auch kurz: Behandlungsvorrichtung) weist wenigstens einen extrakorporalen Blutkreislauf mit einem Leitungsinneren auf. Ferner ist sie versehen mit wenigstens einer an oder in dem extrakorporalen Blutkreislauf angeordneten Blutpumpe zum Fördern von Blut innerhalb des Leitungsinneren des extrakorporalen Blutkreislaufs. Zudem weist sie wenigstens eine Fördereinrichtung, etwa eine Substituat- oder Dialysierflüssigkeitspumpe, auf, welche vorgesehen ist zum Einbringen von Substituat oder Dialysierflüssigkeit in die arterielle Blutentnahmeleitung. Weiter weist sie wenigstens eine erfindungsgemäße Steuerungs- und/oder Regelungseinrichtung auf.

Ein erfindungsgemäßes, insbesondere digitales, insbesondere nicht-flüchtiges, Speichermedium (hier auch als Träger bezeichnet), insbesondere in Form von Diskette, RAM, ROM, CD, Festplatte, DVD, USB-Stick, Flashcard, SD-Karte oder EPROM, insbesondere mit elektronisch oder optisch auslesbaren Steuersignalen, kann derart konfiguriert sein, um eine Steuerungs- und/oder Regelungseinrichtung zu einer erfindungsgemäßen Steuerungs- und/oder Regelungseinrichtung zu konfigurieren, mit welcher das oben beschriebene Verfahren bewirkt werden kann.

Dabei können alle, einige oder manche der maschinell durchführbaren Schritte dieses Verfahrens veranlasst werden.

Ein erfindungsgemäßes Computerprogramm-Produkt weist einen volatilen, flüchtigen oder auf einem maschinenlesbaren Träger gespeicherten Programmcode auf, durch den eine Steuerungs- und/oder Regelungseinrichtung zu einer erfindungsgemäßen Steuerungs- und/oder Regelungseinrichtung konfiguriert wird, mit welcher das oben beschriebene Verfahren bewirkt werden kann.

Dabei können alle, einige oder manche der maschinell durchführbaren Schritte dieses Verfahrens veranlasst werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte, ein EPROM und dergleichen sein.

Ein erfindungsgemäßes Computerprogramm weist einen Programmcode auf, durch den eine Steuerungs- und/oder Regelungseinrichtung zu einer erfindungsgemäßen Steuerungs- und/oder Regelungseinrichtung konfiguriert wird, mit welcher das oben beschriebene Verfahren bewirkt werden kann.

Dabei können alle, einige oder manche der maschinell durchführbaren Schritte dieses Verfahrens veranlasst werden.

Unter einem Computerprogramm-Produkt kann erfindungsgemäß beispielsweise ein auf einem Träger gespeichertes Computerprogramm, ein Embedded System als umfassendes System mit einem Computerprogramm (z. B. ein elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Unter einem Computerprogramm kann erfindungsgemäß beispielsweise ein physikalisches, vertriebsfähiges Software-Produkt verstanden werden, welches ein Programm aufweist.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von "programmiert" oder "konfiguriert" die Rede ist, so ist auch offenbart, diese Begriffe gegeneinander auszutauschen.

Wann immer hierin eine Eignung oder ein Verfahrensschritt genannt ist, umfasst die vorliegende Erfindung auch eine entsprechende Programmierung oder Konfigurierung einer geeigneten Vorrichtung oder eines Abschnitts hiervon sowie derart programmierte Vorrichtungen.

Vorteilhafte Weiterentwicklungen der vorliegenden Erfindung sind jeweils Gegenstand von Unteransprüchen und Ausführungsformen.

Wenn hierin von einer Ausführungsform die Rede ist, so stellt dies eine erfindungsgemäße, beispielhafte Ausführungsform dar.

Wenn hierin von Zeit, Zeitdauer oder Ähnlichem die Rede ist, so ist das Vorhandensein von entsprechenden, geeigneten Zeitmesseinrichtungen mit umfasst.

Wenn hierin von der Beendigung einer Blutbehandlung oder einer Blutbehandlungssitzung die Rede ist, dann kann sich dies auf den Zeitpunkt beziehen, zu welchem der Blutbehandlungseinrichtung, z. B. dem Blutfilter, kein Blut des Patienten mehr zugeführt wird, oder zu welchem dem Patienten kein Blut mehr entnommen wird.

Wenn hierin vom Aufbauen eines erhöhten Drucks mittels der zum Einbringen verwendeten Pumpe oder Fördereinrichtung die Rede ist, so kann hierunter ein Erhöhen des bis zum Beginn des Aufbauens herrschenden Drucks verstanden werden. Das Aufbauen eines erhöhten Drucks kann also ein Erhöhen von Druck sein. Unter einem Aufbauen von Druck, oder von einem erhöhten Druck, wird somit vorzugsweise kein Absenken von Druck und auch kein Beibehalten eines unveränderten Drucks verstanden.

Erfindungsgemäße Ausführungsformen können eines oder mehrere der oben und/oder im Folgenden genannten Merkmale in jeder technisch möglichen Kombination aufweisen.

In manchen Ausführungsformen der Steuerungs- und/oder Regelungseinrichtung liegt der erste vorbestimmte Wert für den Druck zum Öffnen des Ventils der medizinischen Behandlungsvorrichtung z. B. zwischen 1 und 2 bar.

In anderen Ausführungsformen der Steuerungs- und/oder Regelungseinrichtung wird das Ventil der medizinischen Behandlungsvorrichtung nach einer vorbestimmten ersten Zeitdauer geöffnet, diese liegt vorzugsweise zwischen 2 Sekunden (sec) und 5 Sekunden (sec).

In manchen Ausführungsformen der Steuerungs- und/oder Regelungseinrichtung sinkt der Druck, bei dem das Ventil der medizinischen Behandlungsvorrichtung wieder geschlossen wird, auf oder unter einen zweiten vorbestimmten Wert, z. B. zwischen 0 und 0,5 bar.

In anderen Ausführungsformen der Steuerungs- und/oder Regelungseinrichtung wird das Ventil der medizinischen Behandlungsvorrichtung nach einer vorbestimmten zweiten Zeitdauer geschlossen, diese liegt vorzugsweise zwischen 1 sec und 3 sec.

Beim Einbringen von Substituat oder Dialysierflüssigkeit in den extrakorporalen Blutkreislauf und/oder Aufbauen eines erhöhten Drucks wird aufgrund der Compliance des Schlauchsystems, die das Speichern von Energie durch elastische Verformung von Schlauchabschnitten ermöglicht, Energie gespeichert. Die Compliance wirkt daher wie ein Energiespeicher. Beim Öffnen des oben genannten Ventils wird die aufgrund der Compliance gespeicherte Energie entladen oder freigesetzt. Dies kann zum möglichst schlagartigen Freisetzen des Fluids oder zur erhöhten Strömungsgeschwindigkeit beim Öffnen des Ventils vorteilhaft beitragen.

In einigen Ausführungsformen wird beim Ausführen des Verfahrens mittels der erfindungsgemäßen Steuerungs- und/oder Regelungseinrichtung ein Abschnitt, z. B. ein freies Ende oder ein patientenseitiges Ende, der arteriellen Blutentnahmeleitung in Fluidverbindung mit einem weiteren Abschnitt der venösen Blutrückgabeleitung gebracht. Der weitere Abschnitt kann auf einer Blutkassette vorliegen. Er kann mit einem Verbindungsabschnitt versehen und vorbereitet sein, um z. B. mit einem optionalen Verbinder (z. B. Luer-Verbinder, Bajonett-Verbinder) der arteriellen Blutentnahmeleitung verbunden zu werden.

Diese Verbindung dient der Entleerung des arteriellen Abschnitts des extrakorporalen Blutkreislaufs in die venöse Blutrückgabeleitung, z. B. stromauf der Zugabestelle für Substituat (Prädilution).

Ein bestehender oder angestrebter Druck kann in der arteriellen Leitung optional auch durch andere Maßnahmen, z. B. mittels einer okkludierenden Pumpe, beispielsweise einer Blutpumpe, oder mittels Schließen einer arteriellen Patientenschlauchklemme aufrechterhalten werden und damit ein Abbau des Drucks über die arterielle Seite des Blutkreislaufs verhindert werden.

In manchen Ausführungsformen weist der extrakorporale Blutkreislauf der Behandlungsvorrichtung, auf die die erfindungsgemäße Steuerungs- und/oder Regelungseinrichtung einwirkt, eine venöse Luftabscheidekammer oder Blutkammer (welche nicht Bestandteil des Blutfilters ist) auf. In einigen dieser Ausführungsformen beginnt das oben genannte Verfahren mit den Schritten a) bis f) erst, nachdem festgestellt wurde, z. B. mittels geeigneter Sensoren wie optischen Sensoren, dass die venöse Luftabscheidekammer nicht vollständig oder nicht über ein vorbestimmtes Maß hinaus mit Flüssigkeit, insbesondere Blut, gefüllt ist.

In einigen Ausführungsformen weist der extrakorporale Blutkreislauf sowohl eine venöse Luftabscheidekammer als auch eine Single-Needle-Kammer auf. Das Ventil zum Herstellen einer Fluidverbindung ist hierbei optional zwischen der Single-Needle-Kammer und der venösen Luftabscheidekammer angeordnet. Diese beiden Kammern und das Ventil können wiederum auf einer Blutkassette angeordnet oder Teil hiervon sein.

In manchen Ausführungsformen ist statt der Single-Needle-Kammer ein beliebiger Ausgleichsbehälter angeordnet oder wird durch Öffnen des Ventils fluidtechnisch eröffnet oder eingebunden.

In einigen Ausführungsformen wird nach oder im Anschluss an Schritt f) die geringe Flüssigkeitsmenge aus dem Ausgleichsbehälter aktiv ausgetrieben, z. B. mittels eines in manchen Dialysemaschinen wie der 6008 von Fresenius, Deutschland, ohnehin vorhandenen Single-Needle-Kompressors.

In manchen Ausführungsformen werden die Schritte a) bis f) des Verfahrens, das durch die erfindungsgemäße Steuerungs- und/oder Regelungseinrichtung bewirkt wird, mehrfach hintereinander ausgeführt.

Für eine hohe Wirksamkeit können die Schritte b) bis e) während der Dauer der Blutrückgabe, typischerweise innerhalb von drei bis vier Minuten, in optional bestimmten Abständen, mehrfach, vorzugsweise fünf bis 15 Mal oder öfter, wiederholt werden.

In einigen Ausführungsformen ist wenigstens ein Abschnitt des extrakorporalen Blutkreislaufs der erfindungsgemäßen medizinischen Behandlungsvorrichtung Teil einer Blutkassette.

Eine solche Blutkassette ist beispielsweise in der WO 2010/121819 A1 der Anmelderin der vorliegenden Anmeldung beschrieben. Ihr diesbezüglicher Inhalt wird hiermit mittels Verweises vollumfänglich zum Gegenstand auch der vorliegenden Anmeldung gemacht.

Eine solche Blutkassette kann wenigstens einen Gehäusekörper, vorzugsweise aus einem Hart-Kunststoff gefertigt, aufweisen, mit wenigstens einer, zwei oder mehreren in den Gehäusekörper integrierten Kammer(n) zum Aufnehmen medizinischer Fluide, wenigstens einem in den Gehäusekörper integrierten Kanal zum Aufnehmen und/oder Führen eines medizinischen Fluids, und wenigstens einer in den Gehäusekörper ganz oder teilweise integrierten Ventileinrichtung zum Steuern oder Regeln eines die Blutkassette durchströmenden Fluids.

Eine solche Blutkassette kann wenigstens eine Single-Needle-Kammer zum Aufnehmen von Blut stromab des Blutfilters aufweisen. Eine solche Single-Needle-Kammer kann wenigstens ein Single-Needle-Ventil umfassen oder hiermit in Fluidverbindung stehen, welches den Ein- und/oder Ausstrom vom Blut in die Single-Needle-Kammer steuert oder regelt. Beispiele für eine Single-Needle-Kammer und ein Single-Needle-Ventil sind beispielsweise in der WO 2010/121819 A1 beschrieben, deren diesbezüglicher Inhalt hiermit mittels Verweises vollumfänglich zum Gegenstand auch der vorliegenden Anmeldung gemacht wird.

Eine solche Blutkassette kann an wenigstens einer ihrer Oberflächen eine Abdeckungseinrichtung, z. B. eine Folie, aufweisen, welche vorzugsweise Teil wenigstens einer integrierten Ventileinrichtung ist.

Bei einer solchen Blutkassette kann die Abdeckungseinrichtung, z. B. als Folie, wenigstens in einem Abschnitt mit dem Gehäusekörper kraft- und/oder form- und/oder stoffschlüssig verbunden sein.

Bei einer solchen Blutkassette kann die Abdeckungseinrichtung mittels wenigstens einer umlaufenden Schweißnaht mit dem Gehäusekörper verbunden sein.

Die optionale Single-Needle-Kammer kann durch entsprechendes Schalten des optionalen Single-Needle-Ventils, welches die Single-Needle-Kammer vorzugsweise von weiteren, insbesondere von allen weiteren blutführenden Strukturen, trennt, in den Blutkreislauf eingeschaltet werden oder nicht. Wird die Single-Needle-Kammer durch Öffnen des Single-Needle-Ventils für Blut oder ein anderes Fluid, das aus dem venösen Abschnitt stromabwärts des Blutfilters strömt, zugänglich, und wird zugleich beispielsweise mittels Schließens der venösen Patientenschlauchklemme ein Vorbeiströmen des Bluts oder Fluids an der Single-Needle-Kammer verhindert, so kann Blut zunächst in der Single-Needle-Kammer gepuffert oder gespeichert werden, bevor dieses nach Öffnen der venösen Patientenschlauchklemme die Single-Needle-Kammer in Richtung zur venösen Patientenschlauchklemme wieder verlässt.

Der verwendete Blutfilter ist in manchen Ausführungsformen ein Hämodialysator.

In einigen beispielhaften erfindungsgemäßen Ausführungsformen umfasst das Verfahren das Verdrängen des Fluids aus der Blutkammer durch Einbringen von Substituat oder Dialysierflüssigkeit in die arterielle Blutentnahmeleitung mit einem Fluss des Substituats oder der Dialysierflüssigkeit, welcher, zumindest phasenweise, über 150 ml/min, über 200 ml/min, über 250 ml/min, über 300 ml/min, über 350 ml/min, über 400 ml/min, über 450 ml/min, über 500 ml/min, über 550 ml/min und/oder gleich oder unter 600 ml/min liegt.

Die Reihenfolge der Schritte a) bis f) kann optional bindend sein, insbesondere folgt Schritt f) vorzugsweise erst auf Schritt e).

In einigen Ausführungsformen dauert eine Abfolge der Schritte a) bis f) vorzugsweise maximal 5 bis 10 Sekunden. Schaltzeiten für Ventile und Klemmen können hier inklusive sein.

In manchen Ausführungsformen erfolgt während einer Abfolge der Schritte a) bis f) keine Erhöhung der Fördergeschwindigkeit, mit der Substituat oder Dialysierflüssigkeit mittels der entsprechenden Fördereinrichtung gefördert wird. Eine solche Erhöhung, die in diesen Ausführungsformen allerdings unterbleibt, könnte dann gegeben sein, wenn sie mittels Flussmesser stromauf einer Substituat-Zugabestelle messbar wäre. Sie könnte auch dann gegeben sein, wenn sich eine Einstellung der Förderrate oder -geschwindigkeit mittels Steuervorrichtung der Fördereinrichtung geändert hätte. Die eingestellte oder angestrebte Förderrate oder -geschwindigkeit bleibt also unverändert und/oder konstant.

Vorzugsweise wird vor Start der Blutrückgabe, innerhalb welcher dieses Verfahren abläuft, sichergestellt, dass die venöse Luftabscheidekammer nicht vollständig mit Blut gefüllt ist.

In manchen Ausführungsformen wird während einer Abfolge der Schritte a) bis f) nicht aktiv in das Äußere, z. B. in den Ausgleichsbehälter, hineingefördert. Ein Füllen des Ausgleichsbehälters mit Flüssigkeit erfolgt in diesen Ausführungsformen - vorzugsweise allein oder im Wesentlichen allein - aufgrund der Druckentlastung bei Öffnen des Ventils.

Vorzugsweise wird nach Start der Blutrückgabe an den Patienten, beispielsweise durch eine Pumpe, Flüssigkeit in Richtung des Dialysators gefördert, vorzugsweise ohne dass die zur Einbringung von Substituat oder Dialysierflüssigkeit verwendete Pumpe während der Schritte a) bis f) in ihrer Fördertätigkeit gestoppt oder unterbrochen wird.

In einigen beispielhaften erfindungsgemäßen Ausführungsformen wird die Verbindung zwischen arterieller Blutentnahmeleitung und venöser Blutrückgabeleitung insbesondere ausschließlich zum Zwecke der Entleerung des Blutfilters erzeugt.

Eine "Blutbehandlungssitzung" kann beispielsweise eine Behandlungseinheit mittels Hämodialyse, Hämofiltration, Hämodiafiltration und/oder eines Zellseparationsverfahrens und auf die Behandlung und/oder Reinigung von Blut gerichtet sein. Zum Durchführen einer solchen Blutbehandlung wird eine geeignete Blutbehandlungsvorrichtung verwendet.

Fördereinrichtungen zum Einbringen von Substituat oder Dialysierflüssigkeit, wie hierin genannt, schließen Membranpumpen, Schlauchpumpen, Rollenpumpen usw. ein. Die Blutpumpe, eine Substituatpumpe und/oder eine Dialysierflüssigkeitspumpe können z. B. als Schlauchpumpe oder Rollenpumpe ausgeführt sein. Es kann aber auch ein anderer Pumpentyp, z. B. eine Membranpumpe, insbesondere eine hochgenau dosierende Membranpumpe, eingesetzt werden.

Bei einer Fördereinrichtung für Dialysierflüssigkeit oder Substituat kann es sich um eine "zweite" Fördereinrichtung handeln, also eine Fördereinrichtung, welche nicht mit der Blutpumpe identisch ist. Die Blutpumpe kann jedoch auch derart ausgestaltet sein, dass sie sowohl die für eine Blutpumpe typische Funktion ausführt als auch die Funktion des Einbringens von Substituat in das Leitungsinnere und/oder das Fördern von Leitungsinhalt erfüllt. Wenn im Folgenden allein der besseren Lesbarkeit wegen von einer Fördereinrichtung zum Einbringen von Substituat oder Dialysierflüssigkeit die Rede ist, so ist darunter die Blutpumpe oder eine sich hiervon unterscheidende Fördereinrichtung zu verstehen. Beide Varianten sind gleichermaßen von der vorliegenden Erfindung umfasst.

Das "Einbringen von Substituat oder Dialysierflüssigkeit in das Leitungsinnere des extrakorporalen Blutkreislaufs" erfolgt, wie vorstehend ausgeführt, in einigen erfindungsgemäßen Ausführungsformen durch Betreiben der Blutpumpe und/oder der Fördereinrichtung.

Die Blutpumpe kann Substituat fördern, indem sie selbiges aus einer Zuleitung zu einem Behälter für das Substituat ansaugt, wobei die Zuleitung stromaufwärts der Saugseite der Blutpumpe in den extrakorporalen Blutkreislauf mündet. Hierzu kann beispielsweise eine im arteriellen Zweig des extrakorporalen Blutkreislaufs vorgesehene Mündung mit Schlauchklemme vorgesehen sein.

Ist vorgesehen, dass die Blutpumpe sowohl Blut als auch Substituat in den extrakorporalen Blutkreislauf einbringt und fördert, kann das erfindungsgemäße Verfahren mit nur einer Pumpe durchgeführt werden. Obwohl eine solche weiter bevorzugte Ausführungsform von der vorliegenden Erfindung umfasst ist, werden im Folgenden Ausführungsformen beschrieben, bei denen eine Blutpumpe und eine zweite Fördereinrichtung eingesetzt werden. Die folgende Beschreibung soll ein Verständnis der der vorliegenden Erfindung zugrunde liegenden Prinzipien und Funktionen der einzelnen Komponenten vereinfachen. Dabei kann das Substituat oder die Dialysierflüssigkeit mit der Blutpumpe und/oder der Fördereinrichtung, etwa der Substituatpumpe, gefördert werden. Sie können einzeln oder gemeinsam zum Fördern und/oder Aufbauen von Druck wie hierin beschrieben verwendet werden.

Ein "Substituat" kann dabei beispielsweise jedes zur Verwendung bei einer Blutbehandlung, wie z. B. einer Hämodiafiltration, allgemein bekannte Substituat oder Dialysierflüssigkeit sein, vorzugsweise eine bereits während der Blutbehandlungssitzung verwendete und somit über eine Fluidverbindung bereits in den extrakorporalen Blutkreislauf eingebrachte oder einbringbare Lösung oder isotone Kochsalzlösung, wie z. B. eine 0,9%-ige NaCl-Lösung.

Der "Abschnitt des extrakorporalen Blutkreislaufs" kann ein arterieller und/oder venöser Abschnitt des extrakorporalen Blutkreislaufs sein. Der "arterielle Abschnitt" bezeichnet einen Abschnitt des extrakorporalen Blutkreislaufs, durch den das Blut aus dem Gefäßsystem des Patienten in Richtung zur Blutbehandlungseinrichtung oder zum Blutfilter strömt. Der "venöse Abschnitt" bezeichnet den Abschnitt des extrakorporalen Blutkreislaufs, durch den das Blut aus der Blutbehandlungseinrichtung oder aus dem Blutfilter zurück zum Gefäßsystem des Patienten strömt.

Eine "Zugabestelle des extrakorporalen Blutkreislaufs für Substituat in das Leitungsinnere des extrakorporalen Blutkreislaufs" kann in dem arteriellen und/oder dem venösen Abschnitt des extrakorporalen Blutkreislaufs angeordnet sein. Bevorzugt ist die "Zugabestelle" in einem Abschnitt des extrakorporalen Blutkreislaufs angeordnet, der stromauf der Blutbehandlungseinrichtung, beispielsweise stromauf des Blutfilters, durchströmt wird.

Geeignete Beispiele für eine Zugabestelle schließen ein Öffnungs-/Verschlussventil, einen Absperrhahn, eine zuschaltbare Nebenleitung eines verzweigten Abschnitts des extrakorporalen Blutkreislaufs usw. ein.

Das "Rückführen von Blut in das Gefäßsystem des Patienten" kann erfolgen, wenn ein Ende des extrakorporalen Blutkreislaufs, wie beispielsweise das Ende des venösen Abschnitts, z. B. die venöse Konnektionsnadel, mit dem Gefäßsystem des Patienten konnektiert ist. Diese Verbindung kann nach Beenden der Blutbehandlungssitzung beibehalten oder erneut hergestellt werden.

Da mit der erfindungsgemäßen Behandlungsvorrichtung das vorstehend beschriebene Verfahren durchführbar ist, wird zur Vermeidung von Wiederholungen auf die vorstehend beschriebenen Ausführungen derselben verwiesen.

Eine Weiterentwicklung der erfindungsgemäßen Behandlungsvorrichtung sieht das Anordnen wenigstens einer Erfassungseinrichtung zum Erfassen wenigstens einer Änderung des Inhalts des Leitungsinneren des extrakorporalen Blutkreislaufs oder einer Eigenschaft des Inhalts in einem Abschnitt des extrakorporalen Blutkreislaufs vor. Bei einer Eigenschaft des Inhalts kann es sich um eine Zusammensetzung, eine physikalische, chemische oder biologische Größe, beispielsweise eine Transparenz, einen pH-Wert und vieles dergleichen mehr handeln. Eine derartige Erfassungseinrichtung kann der vorstehend beschriebenen entsprechen, so dass zur Vermeidung von Wiederholungen auf deren obige Beschreibung verwiesen wird.

Eine Behandlungsvorrichtung gemäß der vorliegenden Erfindung kann, ohne darauf beschränkt zu sein, zum Durchführen einer Hämodialyse, einer Hämofiltration, einer Hämodiafiltration und von Separationsverfahren geeignet und/oder konfiguriert sein.

Mittels mancher erfindungsgemäßen Ausführungsformen können eine oder mehrere der hierin genannten Vorteile erzielbar sein.

So kann die vorliegende Erfindung in bestimmten erfindungsgemäßen Ausführungsformen zu einer Verbesserung der Effektivität der Blutrückgabe beitragen.

Ein erfindungsgemäß in einigen Ausführungsformen erzielbarer Vorteil besteht darin, dass einem Kontaminationsrisiko bei der weiteren Handhabung oder der Entsorgung des Blutfilters und/oder des extrakorporalen Blutkreislaufs vorgebeugt oder dieses verringert werden kann, da nach Abschluss des erfindungsgemäßen Verfahrens kein Blut mehr im Blutfilter verbleibt oder dessen Menge merklich verringert wurde.

Die Entleerung wenigstens des Blutfilters, insbesondere von Blut, kann in bestimmten Ausführungsformen der Erfindung ohne wesentliche oder ohne jede Interaktion des Benutzers, z. B. des Arztes, erfolgen, jedenfalls bis zur Entnahme des Blutfilters von der Behandlungsvorrichtung. Hiermit einher geht, dass die Fehleranfälligkeit niedrig ist. Zudem hat der Benutzer während der Entleerung Zeit für andere Tätigkeiten. Dies trägt zur Arbeitsentlastung und zur Zeiteinsparung insgesamt bei.

An der Innenwand der Membrankapillaren anhaftende Blutbestandteile werden aufgrund der erhöhten Strömungsgeschwindigkeit und des damit erhöhten Eintrags an Bewegungsenergie und der damit verbundenen Reibung verbessert abgelöst.

Die vorliegende Erfindung kann ferner in vorteilhafter Weise auch zum sicheren Entfernen auch von Blut aus dem extrakorporalen Blutkreislauf für eine Behandlungsvorrichtung zur extrakorporalen Blutbehandlung eines Patienten nach Beenden einer Blutbehandlungssitzung eingesetzt werden: Da sich nach Beenden der Blutbehandlungssitzung Substituat im Leitungsinneren des extrakorporalen Blutkreislaufs befindet, kann das im Leitungsinneren des extrakorporalen Blutkreislaufs vorhandene Blut aus dem extrakorporalen Blutkreislauf entfernt werden. Dies kann ohne Gefahr erfolgen, Luft in den Körper des Patienten einzubringen.

Daneben kann es auch möglich sein, eine Schaumbildung, z. B. am Ausgang des Blutfilters, zu minimieren, indem dort Substituat, also insbesondere eine Flüssigkeit, nicht aber ein Gas (z. B. Luft) zum Verdrängen von Blut oder zum Entleeren eingesetzt wird.

Da das Verfahren, das durch die erfindungsgemäße Steuerungs- und/oder Regelungseinrichtung bewirkt wird, direkt nach Beenden einer Blutbehandlungssitzung durchgeführt werden kann, ist es einfach und leicht durchzuführen und erfordert keine technisch aufwändigen, zeit- und/oder kostenintensiven Schritte.

Das Verfahren, das durch die erfindungsgemäße Steuerungs- und/oder Regelungseinrichtung bewirkt wird, kann in vorteilhafter Weise mit der bei einer Blutbehandlung ohnehin eingesetzten oder vorliegenden Substituats- oder Dialysierflüssigkeit, wie beispielsweise einer isotonen Kochsalzlösung, z. B. einer 0,9%-igen NaCl-Lösung, durchgeführt werden. Dies trägt wiederum in vorteilhafter Weise zu einer Kosten- und Zeitersparnis bei.

Ferner kann das Verfahren, das durch die erfindungsgemäße Steuerungs- und/oder Regelungseinrichtung bewirkt wird, ein Entfernen von Blut aus dem arteriellen Abschnitt des extrakorporalen Blutkreislaufs und insbesondere aus der arteriellen Blutentnahmeleitung und ein Rückführen desselben in das Gefäßsystem des Patienten ermöglichen. Es kann somit in vorteilhafter Weise der Schritt umgangen werden, das in der Blutentnahmeleitung befindliche Blut mit Hilfe z. B. einer mit Kochsalzlösung aufgezogenen Spritze retrograd herauszudrücken.

Das Verfahren, das durch die erfindungsgemäße Steuerungs- und/oder Regelungseinrichtung bewirkt wird, kann somit den Vorteil bieten, das im Leitungsinneren eines extrakorporalen Blutkreislaufs nach dessen Verwendung bei einer Blutbehandlung vorhandene Blut im Wesentlichen vollständig für den Patienten zurückzugewinnen.

Die entstehenden Flussspitzen können vorteilhafterweise zum Ablösen von Blut von den Oberflächen des extrakorporalen Blutkreislaufs beitragen. Durch den intermittierend erhöhten Druck lösen sich Blutreste von Strukturen wie dem Blutfilter.

Diese können dem Patienten in Folge ebenfalls reinfundiert werden.

Außerdem kann durch das automatisierte Bewirken der Flussspitzen die Notwendigkeit eines manuellen Eingriffs durch den Anwender umgangen und daraus folgend die Effizienz des Verfahrens gesteigert werden.

Erfindungsgemäß gibt es keine Flussspitzen, die sich über den extrakorporalen Blutkreislauf hinaus bis in das Gefäßsystem des Patienten fortsetzen. Durch die druckkontrollierte Steuerung des Ablaufs wird dies vorteilhaft vermieden und das Gefäßsystem des Patienten geschont.

Vorteilhafterweise wird durch das gezielte Öffnen des Ventils, z. B. zwischen venöser Luftabscheidekammer und Ausgleichsbehälter, und durch das damit verbundene Entweichen von Druck automatisch einer unerwünschten Druckentwicklung in das Gefäßsystem des Patienten hinein, die schädlich für den Patienten sein kann, entgegengewirkt.

Da erfindungsgemäß nur eine sehr geringe Menge an Flüssigkeit in einen Ausgleichsbehälter gelangt, ist ein sich anschließendes, aktives Austreiben dieser Flüssigkeit aus dem Ausgleichsbehälter nicht erforderlich. Dies erlaubt es, auf eine Pumpe oder andere Fördereinrichtungen hierzu zu verzichten.

Im Folgenden wird das erfindungsgemäße Verfahren anhand bevorzugter Ausführungsformen desselben unter Bezugnahme auf die angehängte Zeichnung beschrieben. In der Zeichnung gilt:

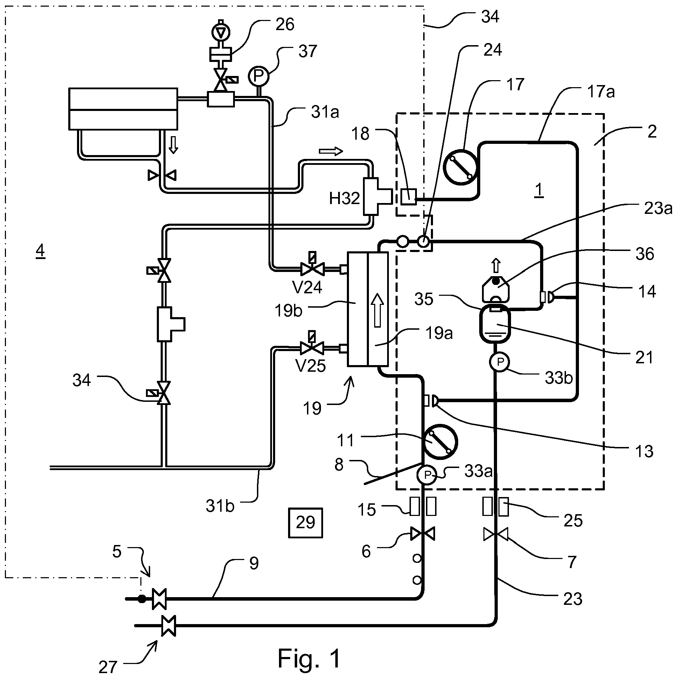

- **Fig. 1**: zeigt schematisch vereinfacht Abschnitte einer erfindungsgemäßen, medizinischen Behandlungsvorrichtung mit einer Blutkassette zur Durchführung des Verfahrens, gesteuert und/oder geregelt durch eine erfindungsgemäße Steuerungs- und/oder Regelungseinrichtung in einer beispielhaften Ausführungsform.

**Fig. 1** zeigt einen extrakorporalen Blutkreislauf 1, welcher zu einer Behandlung mittels Double-Needle-Zugangs, oder unter Verwendung, z. B. eines zusätzlichen Y-Verbinders, mittels Single-Needle-Zugangs, mit dem Gefäßsystem des nicht dargestellten Patienten verbunden werden würde. Der Blutkreislauf 1 liegt optional in Abschnitten hiervon in oder auf einer Blutkassette 2 vor. Diese Blutkassette 2 ist ausgestaltet, um auch bei anderen Behandlungsarten, beispielsweise einer Single-Needle-Behandlung, Verwendung zu finden.

Pumpen, Aktoren und/oder Ventile im Bereich des Blutkreislaufs 1 sind mit einer erfindungsgemäßen Behandlungsvorrichtung 4 bzw. mit einer erfindungsgemäßen Steuerungs- und/oder Regelungseinrichtung 29 verbunden.

Der Blutkreislauf 1 weist eine arterielle Patientenschlauchklemme 6 und eine arterielle Konnektionsnadel 5 (als Beispiel einer Zugangseinrichtung) eines arteriellen Abschnitts oder einer arteriellen Patientenleitung oder Blutentnahmeleitung 9 auf. Der Blutkreislauf 1 weist ferner eine venöse Patientenschlauchklemme 7 und eine venöse Konnektionsnadel 27 (als Beispiel einer weiteren oder zweiten Zugangseinrichtung) eines venösen Abschnitts oder einer venösen Patientenleitung oder Blutrückgabeleitung 23 auf.

Eine Blutpumpe 11 ist im arteriellen Abschnitt 9 vorgesehen, eine Substituatpumpe 17 ist mit einer Substituatleitung 17a verbunden. Die Substituatleitung 17a kann mittels eines, vorzugsweise automatischen, Substituatports 18 mit einer Substituatquelle verbunden werden. Mittels der Substituatpumpe 17 kann Substituat per Prädilution oder per Postdilution über zugehörige Leitungen 13 bzw. 14 in Leitungsabschnitte, beispielsweise in den arteriellen Abschnitt 9 bzw. in einen venösen Abschnitt 23a (zwischen einer Blutkammer 19a eines Blutfilters 19 und einer venösen Luftabscheidekammer 21) des Blutkreislaufs 1 eingebracht werden.

Der Blutfilter 19 weist die mit dem arteriellen Abschnitt 9 und mit dem venösen Abschnitt 23 verbundene Blutkammer 19a auf. Eine Dialysatkammer 19b des Blutfilters 19 ist mit einer zur Dialysatkammer 19b führenden Dialysierflüssigkeitszulaufleitung 31a und einer von der Dialysatkammer 19b wegführenden Dialysatablaufleitung 31b verbunden.

Die Dialysierflüssigkeitszulaufleitung 31a weist optional ein Ventil V24 auf, mittels welchem der Fluss innerhalb der Dialysierflüssigkeitszulaufleitung 31a unterbunden werden kann. Die Dialysatablaufleitung 31b weist optional ein Ventil V25 auf, mittels welchem der Fluss innerhalb der Dialysatablaufleitung 31b unterbunden werden kann.

Die Dialysierflüssigkeitszulaufleitung 31a ist ferner optional mittels eines weiteren, maschineninternen Ventils mit einer Druckluftquelle 26 verbunden. Die Druckluftquelle 26 kann Bestandteil der Behandlungsvorrichtung 4 sein oder getrennt hiervon vorliegen. Stromab der Druckluftquelle 26 kann ein Drucksensor 37 vorgesehen sein.

Die in Fig. 1 als Strich-Punkt-Linie angedeutete Fluidverbindung 34 stellt eine Verbindung zwischen dem arteriellen Abschnitt 9 und dem venösen Abschnitt, hier stromauf der Zugabestelle 14 für Substituat (Postdilution), dar. Die Fluidverbindung 34 kann u. a. der einfachen, automatischen Entleerung des arteriellen Abschnitts 9 bzw. der Zuleitung 8 des extrakorporalen Blutkreislaufs dienen. Die Fluidverbindung 34 kann zudem dafür sorgen, dass sich der erfindungsgemäß aufgebaute Druck nicht ungewollt über die arteriellen Abschnitt 9 abbauen kann.

Die Fluidverbindung 34 kann mittels eines werkseitig vorgesehenen Verbindungsabschnitts 24 mit z. B. dem venösen Abschnitt 23 oder 23a erzielt werden. Der Verbindungsabschnitt 24 kann eine Fluidverbindung mit dem venösen Abschnitt 23 oder 23a ermöglichen.

Die Fluidverbindung 34 kann auf einfache Weise erzielt werden, indem der arterielle Abschnitt 9 vom Patienten dekonnektiert und z. B. mittels des optionalen Verbindungsabschnitts 24 mit z. B. dem venösen Abschnitt 23 oder 23a verbunden wird.

Zum Aufrechterhalten des Drucks kommen anstelle (oder ergänzend zu) der Fluidverbindung 34 auch eine oder mehrere andere Maßnahmen in Frage. So kann z. B. mittels einer okkludierenden Pumpe, hier der Blutpumpe 11, oder mittels Schließen der arteriellen Patientenschlauchklemme 6, ein Abbau des Drucks über die arterielle Seite des Blutkreislaufs verhindert werden.

Um nach Ende der Behandlung das Verfahren zum Entleeren des extrakorporalen Blutkreislaufs 1 von Blut, insbesondere das Entleeren der Blutkammer 19a des Blutfilters 19, zu bewirken, können durch die erfindungsgemäße Steuerungs- und/oder Regelungseinrichtung 29 die folgenden Schritte a) bis f) ein- oder mehrfach ausgeführt werden. Dabei wird vorausgesetzt, dass - vorzugsweise automatisch - sichergestellt wurde, dass die venöse Luftabscheidekammer 21 nur zum Teil, also nicht vollständig, mit Blut gefüllt ist.
a) Unterbinden einer Fluidverbindung zwischen der venösen Luftabscheidekammer 21 einerseits und der Single-Needle-Kammer 36 (als Beispiel für die Atmosphäre oder das Äußere) andererseits durch Schließen eines Ventils, hier beispielsweise des Ventils 35;
b) Schließen der venösen Patientenschlauchklemme 7 oder Unterbinden des Flusses entlang der venösen Blutrückgabeleitung 23 stromab des Blutfilters 19 auf andere Weise;
c) Einbringen von Substituat oder Dialysierflüssigkeit in den Blutkreislauf 1, insbesondere in die arterielle Blutentnahmeleitung 9 oder in die venöse Blutrückgabeleitung 23;
d) Messen des in der arteriellen Blutentnahmeleitung 9 und/oder in der venösen Blutrückgabeleitung 23 herrschenden Drucks mittels des Drucksensors (33a, 33b);
e) Herstellen einer Verbindung zwischen der venösen Luftabscheidekammer 21 und der Single-Needle-Kammer 36 des extrakorporalen Blutkreislaufs 1 durch Öffnen des Ventils 35, falls der gemessene Druck einen vorbestimmten ersten Wert erreicht oder übersteigt; und
f) Unterbinden einer Verbindung zwischen der venösen Luftabscheidekammer 21 und der Single-Needle-Kammer 36 des extrakorporalen Blutkreislaufs 1 durch Schließen des Ventils 35 und Öffnen der Patientenschlauchklemme 7, beides nachdem der gemessene Druck auf einen vorbestimmten zweiten Wert abgefallen ist.

In der vorliegenden Ausführungsform (Double-Needle-Verfahren) dient die Single-Needle-Kammer 36 als Entlüftungseinrichtung für die venöse Luftabscheidekammer 21 und repräsentiert daher die Atmosphäre oder das Äußere.

Alternativ ist vorstellbar, dass ein anderes Ventil als das Ventil 35 eine Verbindung zwischen der Atmosphäre/dem Äußeren und dem Inneren des extrakorporalen Blutkreislaufs 1 darstellt und somit durch Öffnen bzw. Schließen dieses Ventils die Verbindung zwischen der Atmosphäre/dem Äußeren und dem Inneren hergestellt bzw. unterbunden wird.

Alternativ, oder ergänzend, kann das Substituat nicht mittels der Substituatpumpe 17, sondern durch Betreiben der Blutpumpe 11 eingebracht werden. Dazu wird die arterielle Patientenschlauchklemme 6 geschlossen und Substituat über eine Zuleitung 8 aus einem Vorratsbehälter für das Substituat in den extrakorporalen Blutkreislauf 1 eingebracht.

Die Steuerungs- und/oder Regelungseinrichtung 29 kann Teil einer Steuerungs- und/oder Regelungseinrichtung der Behandlungsvorrichtung 4 sein.

Die Anordnung der Fig. 1 umfasst einen optionalen Detektor 15 zum Detektieren von Luft und/oder Blut. Die Anordnung der Fig. 1 umfasst ferner einen oder zwei Drucksensoren 33a, 33b an den in Fig. 1 gezeigten Stellen.

Die Single-Needle-Kammer 36 kommt in Fig. 1 als Puffer- und/oder Ausgleichsbehälter zum Einsatz, insbesondere bei oder nach einem Double-Needle-Verfahren, bei welchem der Patient mittels zweier Blutleitungen 9, 23 mit dem extrakorporalen Blutkreislauf 1 verbunden ist. Für den Fachmann ist es erkennbar, dass jeder andere Ausgleichsbehälter ebenfalls in Betracht gezogen werden kann.

Die vorliegende Erfindung ist nicht auf die vorstehend beschriebene Ausführungsform beschränkt, diese dient lediglich der Veranschaulichung. Auch eine Zeitregelung, wie oben beschrieben, kann anstelle oder ergänzend zu der mit Bezug auf Fig. 1 beschriebenen Druckregelung zum Einsatz kommen. Auch ist die Erfindung nicht auf ein Entleeren des Inhalts oder Teile hiervon bei bestehender Konnektierung mit dem Gefäßsystem beschränkt.

### Bezugszeichenliste

- 1: extrakorporaler Blutkreislauf
- 2: Blutkassette
- 4: Behandlungsvorrichtung
- 5: Zugangseinrichtung, beispielsweise arterielle Konnektionsnadel
- 6: arterielle Patientenschlauchklemme
- 7: venöse Patientenschlauchklemme
- 8: Zuleitung
- 9: arterieller Abschnitt oder arterielle Blutentnahmeleitung oder arterielle Patientenleitung
- 11: Blutpumpe
- 13: Zugabestelle für Substituat (Prädilution)
- 14: Zugabestelle für Substituat (Postdilution)
- 15: arterieller Luft-Blut-Detektor
- 17: zweite Fördereinrichtung, beispielsweise eine Substituatpumpe
- 17a: Substituatleitung
- 18: automatischer Substituatport
- 19: Blutfilter
- 19a: Blutkammer
- 19b: Dialysatkammer
- 21: venöse Luftabscheidekammer
- 23: venöser Abschnitt oder venöse Blutrückgabeleitung
- 23a: venöser Abschnitt
- 24: Verbindungsstelle, Verbindungsabschnitt
- 25: venöser Substituat-Blut-Detektor
- 26: Druckluftquelle
- 27: Zugangseinrichtung, beispielsweise venöse Konnektionsnadel
- 29: Steuerungs- oder Regelungseinrichtung
- 31a: Dialysierflüssigkeitszulaufleitung
- 31b: Dialysatablaufleitung
- 33a, b: Drucksensoren
- 34: Fluidverbindung
- 35: Single-Needle-Ventil
- 36: Single-Needle-Kammer
- 37: Drucksensor
- V24: Ventil
- V25: Ventil

## Patentansprüche

1. Steuerungs- und/oder Regelungseinrichtung (29), konfiguriert, um die Durchführung eines Verfahrens zum Entfernen von Blut aus einem zur Blutbehandlung eines Patienten verwendeten Blutfilter (19) oder Blutkreislauf (1) nach Beendigung der Blutbehandlung oder der Blutbehandlungssitzung im Zusammenwirken mit einer Blutbehandlungsvorrichtung (4) zu steuern oder zu regeln,
wobei der Blutkreislauf (1) aufweist:
- einen Blutfilter (19) mit einer Blutkammer (19a) und einer Dialysatkammer (19b), zwischen welchen eine Membran angeordnet ist;
- eine mit der Blutkammer (19a) verbundene arterielle Blutleitung (9) oder Blutentnahmeleitung;
- eine mit der Blutkammer (19a) verbundene venöse Blutleitung (23) oder Blutrückgabeleitung; und
- ein Ventil (35) zum selektiven Herstellen oder Unterbinden einer Fluidverbindung zwischen dem Inneren des Blutkreislaufs (1) und der Atmosphäre oder einem Äußeren des Blutkreislaufs (1);
wobei die Blutbehandlungsvorrichtung (4) aufweist:
- wenigstens eine an oder in dem extrakorporalen Blutkreislauf (1) angeordnete Blutpumpe (11) zum Fördern von Blut innerhalb des Leitungsinneren des extrakorporalen Blutkreislaufs (1);
- wenigstens eine Fördereinrichtung zum Einbringen von Substituat oder Dialysierflüssigkeit (i) in die arterielle Blutleitung (9) oder Blutentnahmeleitung oder (ii) in die venöse Blutleitung (23) oder Blutrückgabeleitung;
- wenigstens eine venöse Patientenschlauchklemme (7) zum Unterbinden eines Flusses durch einen Abschnitt der venösen Blutleitung (23) oder Blutrückgabeleitung;
- wenigstens eine Betätigungseinrichtung zum Betätigen des Ventils (35); und
- wenigstens einen Drucksensor (33a, 33b) zum Messen des in der arteriellen Blutleitung (9) oder Blutentnahmeleitung oder in der venösen Blutleitung (23) oder Blutrückgabeleitung herrschenden Drucks;
wobei das Verfahren die Schritte umfasst:
a) Unterbinden einer Verbindung zwischen der Atmosphäre/dem Äußeren einerseits und einem Inneren des Blutkreislaufs (1) andererseits durch Schließen des Ventils (35);
b) Schließen der venösen Patientenschlauchklemme (7);
c) Einbringen von Substituat oder Dialysierflüssigkeit in den Blutkreislauf (1), insbesondere in die arterielle Blutleitung (9) oder Blutentnahmeleitung oder in die venöse Blutleitung (23) oder Blutrückgabeleitung und/oder Aufbauen eines erhöhten Drucks mittels der zum Einbringen verwendeten Fördereinrichtung;
d) Optional: Messen des in der arteriellen Blutleitung (9) oder Blutentnahmeleitung und/oder in der venösen Blutleitung (23) oder Blutrückgabeleitung herrschenden Drucks mittels des Drucksensors (33a, 33b);
e) Herstellen der Fluidverbindung zwischen der Atmosphäre/dem Äußeren und dem Inneren des Blutkreislaufs (1) durch Öffnen des Ventils (35), falls der gemessene Druck einen vorbestimmten ersten Wert erreicht oder übersteigt, falls gemessen, oder wenn seit Beginn des Einbringens von Substituat oder seit Beginn des Aufbauens von Druck bei geschlossener venöser Patientenschlauchklemme (7) eine vorbestimmte, erste Zeitdauer verstrichen ist; und
f) Unterbinden der Fluidverbindung zwischen der Atmosphäre/dem Äußeren einerseits und dem Inneren des Blutkreislaufs (1) andererseits durch Schließen des Ventils (35) und Öffnen der venösen Patientenschlauchklemme (7), beides wenn der gemessene Druck auf einen vorbestimmten zweiten Wert abgefallen ist, falls gemessen, oder wenn seit dem Öffnen des Ventils (35) in Schritt e) eine vorbestimmte, zweite Zeitdauer verstrichen ist.

2. Steuerungs- und/oder Regelungseinrichtung (29) nach Anspruch 1, wobei der vorbestimmte erste Wert für den Druck zwischen 1 und 2 bar oder die vorbestimmte, erste Zeitdauer zwischen 2 sec und 5 sec liegt.

3. Steuerungs- und/oder Regelungseinrichtung (29) nach Anspruch 1 oder 2, wobei der vorbestimmte zweite Wert zwischen 0 und 0,5 bar oder die vorbestimmte, zweite Zeitdauer zwischen 1 sec und 3 sec liegt.

4. Steuerungs- und/oder Regelungseinrichtung (29) nach einem der Ansprüche 1 bis 3, wobei bei Ausführen des Verfahrens ein freies Ende der arteriellen Blutleitung (9) oder Blutentnahmeleitung in Fluidverbindung (34) mit einem weiteren Abschnitt der venösen Blutleitung (23) oder Blutrückgabeleitung verbunden ist.

5. Steuerungs- und/oder Regelungseinrichtung (29) nach einem der Ansprüche 1 bis 4,
wobei der Blutkreislauf (1) eine venöse Luftabscheidekammer (21) aufweist; und
wobei das Verfahren erst beginnt, nachdem festgestellt wurde, dass die venöse Luftabscheidekammer (21) nicht vollständig oder nicht über ein vorbestimmtes Maß hinaus mit Flüssigkeit, insbesondere Blut, gefüllt ist.

6. Steuerungs- und/oder Regelungseinrichtung (29) nach einem der Ansprüche 1 bis 5, wobei der Blutkreislauf (1) eine venöse Luftabscheidekammer (21) und eine Single-Needle-Kammer (36) aufweist und das Ventil (35) zwischen der Single-Needle-Kammer (36) und der venösen Luftabscheidekammer (21) angeordnet ist.

7. Steuerungs- und/oder Regelungseinrichtung (29) nach einem der Ansprüche 1 bis 6, wobei die Schritte a) bis f) des Verfahrens zum Entfernen von Blut mehrfach hintereinander ausgeführt werden.

8. Medizinische Behandlungsvorrichtung (4) mit
- wenigstens einem extrakorporalen Blutkreislauf (1) mit einem Leitungsinneren;
- wenigstens einer an oder in dem extrakorporalen Blutkreislauf (1) angeordneten Blutpumpe (11) zum Fördern von Blut innerhalb des Leitungsinneren des extrakorporalen Blutkreislaufs (1); und
- wenigstens einer Fördereinrichtung zum Einbringen von Substituat oder Dialysierflüssigkeit in die arterielle Blutleitung (9) oder Blutentnahmeleitung;
**gekennzeichnet durch**
- wenigstens eine Steuerungs- und/oder Regelungseinrichtung (29) nach einem der vorhergehenden Ansprüche.

9. Medizinische Behandlungsvorrichtung (4) nach Anspruch 8, wobei der extrakorporale Blutkreislauf (1) wenigstens abschnittsweise Teil einer Blutkassette (2) ist.

10. Digitales Speichermedium, insbesondere in Form einer Diskette, CD oder DVD oder EPROM, mit elektronisch auslesbaren Steuersignalen, konfiguriert, um eine Steuerungs- und/oder Regelungseinrichtung (29) zu einer Steuerungs- und/oder Regelungseinrichtung (29) gemäß einem der Ansprüche 1 bis 7 zu konfigurieren.

11. Computerprogramm-Produkt mit einem auf einem maschinenlesbaren Träger gespeicherten Programmcode, um eine Steuerungs- und/oder Regelungseinrichtung (29) zu einer Steuerungs- und/oder Regelungseinrichtung (29) gemäß einem der Ansprüche 1 bis 7 zu konfigurieren.

12. Computerprogramm mit einem Programmcode, um eine Steuerungs- und/oder Regelungseinrichtung (29) zu einer Steuerungs- und/oder Regelungseinrichtung (29) gemäß einem der Ansprüche 1 bis 7 zu konfigurieren.

## Claims

1. A control and/or regulating device (29) configured to control or regulate, in interaction with a blood treatment apparatus (4), the execution of a method for removing blood from a blood filter (19) or blood circuit (1) used for the blood treatment of a patient after completion of the blood treatment or the blood treatment session,
wherein the blood circuit (1) comprises:
- a blood filter (19) having a blood chamber (19a) and a dialysate chamber (19b) between which a membrane is arranged;
- an arterial blood line (9) or blood withdrawal line connected to the blood chamber (19a);
- a venous blood line (23) or blood return line connected to the blood chamber (19a);
- a valve (35) for selectively establishing or preventing a fluid connection between the interior of the blood circuit (1) and the atmosphere or an exterior of the blood circuit (1);
wherein the blood treatment apparatus (4) comprises:
- at least one blood pump (11) arranged on or in the extracorporeal blood circuit (1) for conveying blood within the line interior of the extracorporeal blood circuit (1);
- at least one conveying device for introducing susbtituate or dialysis liquid (i) into the arterial blood line (9) or the blood withdrawal line or (ii) into the venous blood line (23) or the blood return line;
- at least one venous patient tube clamp (7) for preventing flow through a section of the venous blood line (23) or of the blood return line;
- at least one actuating device for actuating the valve (35) ; and
- at least one pressure sensor (33a, 33b) for measuring the pressure prevailing in the arterial blood line (9) or blood withdrawal line or in the venous blood line (23) or blood return line;
wherein the method comprises the steps of:
a) preventing a connection between the atmosphere/the exterior on the one hand and an interior of the blood circuit (1) on the other hand by closing the valve (35) ;
b) closing the venous patient tube clamp (7);
c) introducing substituate or dialysis liquid into the blood circuit (1), in particular into the arterial blood line (9) or blood withdrawal line or into the venous blood line (23) or blood return line and/or establishing an increased pressure by means of the conveying device used for introduction;
d) optionally: measuring the pressure prevailing in the arterial blood line (9) or blood withdrawal line and/or in the venous blood line (23) or blood return line by means of the pressure sensor (33a, 33b);
e) establishing the fluid connection between the atmosphere/the exterior and the interior of the blood circuit (1) by opening the valve (35) when the measured pressure reaches or exceeds a predetermined first value, if measured, or when a predetermined first period of time has elapsed since the beginning of the introducing of substituate or since the beginning of the establishing of pressure when the venous patient tube clamp (7) is closed; and
f) preventing the fluid connection between the atmosphere/exterior on the one hand and the interior of the blood circuit (1) on the other hand by closing the valve (35) and opening the venous patient tube clamp (7), both when the measured pressure has dropped to a predetermined second value, if measured, or when a predetermined second period of time has elapsed since the opening of the valve (35) in step e).

2. The control and/or regulating device (29) according to claim 1, wherein the predetermined first value for the pressure is between 1 and 2 bar or the predetermined first period of time is between 2 sec and 5 sec.

3. The control and/or regulating device (29) according to claim 1 or 2, wherein the predetermined second value is between 0 and 0.5 bar or the predetermined second period of time is between 1 sec and 3 sec.

4. The control and/or regulating device (29) according to anyone of claims 1 to 3, wherein a free end of the arterial blood line (9) or blood withdrawal line is connected in fluid communication (34) with a further section of the venous blood line (23) or blood return line, when executing the method.

5. The control and/or regulating device (29) according to anyone of claims 1 to 4,
wherein the blood circuit (1) comprises a venous air separation chamber (21); and
wherein the method only begins after it has been determined that the venous air separation chamber (21) is not completely filled, or not filled beyond a predetermined level, with liquid, in particular blood.

6. The control and/or regulating device (29) according to anyone of claims 1 to 5, wherein the blood circuit (1) comprises a venous air separation chamber (21) and a single-needle chamber (36) and wherein the valve (35) is arranged between the single-needle chamber (36) and the venous air separation chamber (21).

7. The control and/or regulating device (29) according to anyone of claims 1 to 6, wherein steps a) to f) of the method for removing blood are executed several times in succession.

8. A medical treatment apparatus (4) having
- at least one extracorporeal blood circuit (1) with a line interior;
- at least one blood pump (11) arranged on or in the extracorporeal blood circuit (1) for conveying blood within the line interior of the extracorporeal blood circuit (1); and
- at least one conveying device for introducing substituate or dialysis liquid into the arterial blood line (9) or blood withdrawal line;
**characterized by**
- at least one control and/or regulating device (29) according to anyone of the preceding claims.

9. The medical treatment apparatus (4) according to claim 8, wherein the extracorporeal blood circuit (1) is, at least partially, part of a blood cassette (2).

10. A digital storage medium, in particular in the form of a floppy disk, CD or DVD or EPROM, with electronically readable control signals, configured for configuring a control and/or regulating device (29) to a control and/or regulating device (29) according to anyone of claims 1 to 7.

11. A computer program product with a program code saved on a machine-readable carrier for configuring a control and/or regulating device (29) to a control and/or regulating device (29) according to anyone of claims 1 to 7.

12. A computer program with a program code for configuring a control and/or regulating device (29) to a control and/or regulating device (29) according to one of claims 1 to 7.

## Revendications

1. Un dispositif de commande et/ou de régulation (29) configuré pour commander ou réguler, en interaction avec un appareil de traitement du sang (4), l'exécution d'un procédé d'élimination du sang d'un filtre à sang (19) et/ou d'un circuit sanguin (1) utilisé pour le traitement du sang d'un patient après l'achèvement du traitement du sang ou de la séance de traitement du sang,
où le circuit sanguin (1) comprend:
- un filtre à sang (19) ayant une chambre à sang (19a) ainsi qu'une chambre à dialysat (19b) entre lesquelles est agencée une membrane;
- une conduite artérielle de sang (9) ou une conduite de prélèvement sanguin reliée à la chambre à sang (19a);
- une conduite veineuse de sang (23) ou une conduite de retour sanguin reliée à la chambre à sang (19a);
- un clapet (35) pour sélectivement établir ou empêcher une communication fluidique entre l'intérieur du circuit sanguin (1) et l'atmosphère ou un extérieur du circuit sanguin (1);
où l'appareil de traitement du sang (4) comprend:
- au moins une pompe à sang (11) agencée sur ou dans le circuit sanguin extracorporel (1) pour acheminer du sang à l'intérieur de la conduite du circuit sanguin extracorporel (1);
- au moins un dispositif d'acheminement pour introduire du liquide de substitut ou de dialyse (i) dans la conduite artérielle de sang (9) ou la conduite de prélèvement sanguin, ou (ii) dans la conduite veineuse de sang (23) ou la conduite de retour sanguin;
- au moins un collier de serrage de tuyau veineux du patient (7) afin d'empêcher l'écoulement au travers d'une partie de la conduite veineuse de sang (23) ou de la conduite de retour sanguin;
- au moins un dispositif d'actionnement pour actionner le clapet (35); et
- au moins un capteur de pression (33a, 33b) pour mesurer la pression régnant dans la conduite artérielle de sang (9) ou la conduite de prélèvement sanguin, ou dans la conduite veineuse de sang (23) ou la conduite de retour sanguin;
où le procédé comprend les étapes suivantes :
a) l'empêchement d'une communication entre l'atmosphère/ l'extérieur, d'une part, et un intérieur du circuit sanguin (1), d'autre part, par la fermeture du clapet (35);
b) la fermeture du collier de serrage de tuyau veineux du patient (7);
c) l'introduction de liquide de substitut ou de dialyse dans le circuit sanguin (1), notamment dans la conduite artérielle de sang (9) ou la conduite de prélèvement sanguin, ou dans la conduite veineuse de sang (23) ou la conduite de retour sanguin, et/ou l'établissement d'une pression accrue au moyen du dispositif d'acheminement utilisé pour l'introduction;
d) facultatif: la mesure de la pression régnant dans la conduite artérielle de sang (9) ou la conduite de prélèvement sanguin, et/ou dans la conduite veineuse de sang (23) ou la conduite de retour sanguin au moyen du capteur de pression (33a, 33b);
e) l'établissement de la communication fluidique entre l'atmosphère/l'extérieur et l'intérieur du circuit sanguin (1) par l'ouverture du clapet (35) si la pression mesurée atteint, ou dépasse, une première valeur prédéterminée, si mesurée, ou si une première période de temps prédéterminée s'est écoulée depuis le début de l'introduction du substitut ou depuis le début de l'établissement de la pression lorsque le collier de serrage de tuyau veineux du patient (7) est fermé; et
f) l'empêchement d'une communication entre l'atmosphère/ l'extérieur, d'une part, et l'intérieur du circuit sanguin (1), d'autre part, par la fermeture du clapet (35) et l'ouverture du collier de serrage de tuyau veineux du patient (7), tous deux lorsque la pression mesurée a chuté jusqu'à une seconde valeur prédéterminée, si mesurée, ou si une seconde période de temps prédéterminée s'est écoulée depuis l'ouverture du clapet (35) à l'étape e).

2. Le dispositif de commande et/ou de régulation (29) selon la première revendication, où la première valeur prédéterminée de la pression est comprise entre 1 et 2 bars ou la première période de temps prédéterminée est comprise entre 2 et 5 secondes.

3. Le dispositif de commande et/ou de régulation (29) selon la revendication 1 ou 2, où la seconde valeur prédéterminée est comprise entre 0 et 0,5 bars ou la seconde période de temps prédéterminée est comprise entre 1 et 3 secondes.

4. Le dispositif de commande et/ou de régulation (29) selon l'une quelconque des revendications 1 à 3, où une extrémité libre de la conduite artérielle de sang (9) ou de la conduite de prélèvement sanguin est reliée en communication fluidique (34) à une autre partie de la conduite veineuse de sang (23) ou de la conduite de retour sanguin, lors de l'exécution du procédé.

5. Le dispositif de commande et/ou de régulation (29) selon l'une quelconque des revendications 1 à 4,
où le circuit sanguin (1) comprend une chambre de séparation d'air veineuse (21); et
où le procédé ne débute qu'après qu'il ait été déterminé que la chambre de séparation d'air veineuse (21) n'est pas complètement remplie de liquide, notamment de sang, ou n'est pas remplie au-delà d'un niveau prédéterminé.

6. Le dispositif de commande et/ou de régulation (29) selon l'une quelconque des revendications 1 à 5, où le circuit sanguin (1) comprend une chambre de séparation d'air veineuse (21) ainsi qu'une chambre single-needle (36), et où le clapet (35) est agencé entre la chambre single-needle (36) et la chambre de séparation d'air veineuse (21).

7. Le dispositif de commande et/ou de régulation (29) selon l'une quelconque des revendications 1 à 6, où les étapes a) à f) du procédé d'élimination du sang sont exécutées plusieurs fois de suite.

8. Un appareil de traitement médical (4) ayant
- au moins un circuit sanguin extracorporel (1) avec un intérieur de conduite;
- au moins une pompe à sang (11) agencée sur ou dans le circuit sanguin extracorporel (1) pour acheminer le sang à l'intérieur de la conduite du circuit sanguin extracorporel (1); et
- au moins un dispositif d'acheminement pour introduire du liquide de substitut ou de dialyse dans la conduite artérielle de sang (9) ou la conduite de prélèvement sanguin;
**caractérisé par**
- au moins un dispositif de commande et/ou de régulation (29) selon l'une quelconque des revendications précédentes.

9. L'appareil de traitement médical (4) selon la revendication 8, où le circuit sanguin extracorporel (1) fait partie, au moins partiellement, d'une cassette à sang (2).

10. Un support de stockage numérique, notamment sous forme de disquette, de CD ou de DVD ou d'EPROM, comportant des signaux de commande lisibles électroniquement, configuré pour configurer un dispositif de commande et/ou de régulation (29) en un dispositif de commande et/ou de régulation (29) selon l'une quelconque des revendications 1 à 7.

11. Un produit de programme informatique comportant un code de programme enregistré sur un support lisible par machine pour configurer un dispositif de commande et/ou de régulation (29) en un dispositif de commande et/ou de régulation (29) selon l'une quelconque des revendications 1 à 7.

12. Un programme informatique ayant un code de programme pour configurer un dispositif de commande et/ou de régulation (29) en un dispositif de commande et/ou de régulation (29) selon l'une quelconque des revendications 1 à 7.
